# EUROPEAN PATENT APPLICATION

(11) **EP 3 861 948 A1**
(43) Date of publication of application: **11.08.2021**
(21) Application number: 21156253.3
(22) Date of filing: 10.02.2021
(51) Int. Cl.: A61B 17/34, A61B 18/00

(54) **CELLULITE ERADICATION METHODS**

(30) Priority: 10.02.2020 US 202062972253 P
(71) Applicant: Del Vecchio, Daniel A., Boston, MA 02116 (US); Wall, Jr., Simeon, Shreveport, LA 71105 (US)
(72) Inventor: Del Vecchio, Daniel A., Boston, MA 02116 (US); Wall, Jr., Simeon, Shreveport, LA 71105 (US)
(74) Representative: Appleyard Lees IP LLP

(57) **Abstract**

Cellulite eradication methods for substantially eradicating cellulite from a cellulite treatment site on the skin of a patient may include injecting a collagen degrading enzyme into a subcutaneous space and into contact with cellulite band septa in the skin of the patient.

## Description

### FIELD

Illustrative embodiments of the disclosure relate to cellulite treatment methods, such as non-therapeutic (for example cosmetic) cellulite treatment methods. More particularly, illustrative embodiments of the disclosure relate to cellulite eradication methods in which a collagen degrading enzyme may be injected into a subcutaneous space to target, degrade and release cellulite band septa which extend between the fascia below and the dermis above in the subcutaneous space.

### SUMMARY

Illustrative embodiments of the disclosure are generally directed to cellulite eradication methods for substantially eradicating cellulite from a cellulite treatment site on the skin of a patient. An illustrative embodiment of the methods may include targeted release of cellulite band septa in the subcutaneous space by injecting a collagen degrading enzyme into the subcutaneous space and into contact with the cellulite band septa.

In some embodiments, the method may include externally applying negative air pressure to at least one treatment area within a cellulite treatment site and simultaneously injecting a collagen degrading enzyme into the subcutaneous space under the skin at the at least one treatment area while vibrating the collagen degrading enzyme at one or multiple subcutaneous depths at the site of the cellulite band septum or septa.

In some embodiments, the cellulite eradication methods may include forming an incision adjacent to or nearby a cellulite treatment site on the skin of a patient, inserting a cannula of an enzyme injection device through the incision and into an adipose tissue layer in the subcutaneous space in the skin, injecting a collagen degrading enzyme through the cannula into the subcutaneous space and into contact with cellulite band septa extending between the fascia and the dermis in the subcutaneous space, removing the cannula from the incision and closing the incision.

In some embodiments, the cellulite eradication methods may include forming an incision in the skin adjacent or close to a cellulite treatment site on a patient, applying negative air pressure to at least one treatment area within the cellulite treatment site through a suction device, inserting a cannula of an enzyme injection device through the incision and into the adipose tissue layer in the subcutaneous space in the skin, vibrating the cannula, simultaneously injecting a collagen degrading enzyme through the cannula into the subcutaneous space and into contact with the cellulite band septa, removing the cannula from the incision, removing the suction device from the skin and closing the incision.

The present invention provides a cellulite eradication method for substantially eradicating cellulite from a cellulite treatment site on the skin of a patient, comprising: injecting a collagen degrading enzyme into a subcutaneous space and into contact with cellulite band septa in the skin of the patient. The cellulite eradication method may further comprise externally applying negative air pressure to at least one treatment area within the cellulite treatment site simultaneous with injecting the collagen degrading enzyme into the subcutaneous space.

The present invention may further provide a cellulite eradication method for substantially eradicating cellulite from a cellulite treatment site on the skin of a patient, comprising:
forming an incision adjacent or close to the cellulite treatment site on the skin of the patient;
inserting a cannula of an enzyme injection device through the incision and into an adipose tissue layer in the subcutaneous space in the skin;
injecting a collagen degrading enzyme through the cannula into the subcutaneous space into contact with cellulite band septa extending between the fascia and the dermis in the subcutaneous space;
removing the cannula from the incision; and
closing the incision. The cellulite eradication method may further comprise externally applying negative air pressure to at least one treatment area within the cellulite treatment site simultaneous with injecting the collagen degrading enzyme into the subcutaneous space.

The present invention may further provide a cellulite eradication method for substantially eradicating cellulite from a cellulite treatment site on the skin of a patient, comprising:
obtaining an external and internal expansion vibration lipofilling system including an enzyme injection device having a cannula with a cannula tip and a medical suction apparatus having a suction device;
forming an incision adjacent to the cellulite treatment site on the skin of the patient;
externally applying negative air pressure to at least one treatment area within the cellulite treatment site through the suction device of the medical suction apparatus;
inserting the cannula of the enzyme injection device through the incision and into an adipose tissue layer in the subcutaneous space in the skin;
injecting a collagen degrading enzyme through the cannula into the subcutaneous space into contact with cellulite band septa extending between the fascia and the dermis in the subcutaneous space;
removing the cannula from the incision; and
closing the incision.

In the methods of the invention, the step of injecting a collagen degrading enzyme into the subcutaneous space may comprise injecting the collagenase *clostridium histolyticum* into the subdermal space.

The cellulite eradication methods of the invention may further comprise vibrating the collagen degrading enzyme simultaneous with injecting the collagen degrading enzyme into the subcutaneous space, preferably wherein vibrating the collagen degrading enzyme comprises vibrating the collagen degrading enzyme at a reciprocation frequency within the range of about 2,000-10,000 cycles/min, more preferably at a reciprocation frequency of about 7,000 cycles/min.

In the methods of the invention, injecting the collagen degrading enzyme into the subcutaneous space may comprise injecting the collagen degrading enzyme into the subcutaneous space to substantially eradicate cellulite from the skin in at least one of the buttocks, hip, thighs, and lower back of the patient.

The methods of the present invention provide non-therapeutic (for example cosmetic) effects to the patient. In other words, the methods of the present invention are non-therapeutic, i.e. cosmetic, methods. The methods act to improve the bodily appearance of a patient, for example by enhancing the aesthetic appearance of the skin of the patient.

### BRIEF DESCRIPTION OF THE DRAWINGS

Illustrative embodiments of the disclosure will now be described, by way of example, with reference to the accompanying drawings, in which:
FIG. 1 is a block diagram of an illustrative embodiment of an external and internal expansion vibration injection system which is suitable for some implementations of the cellulite eradication methods of the disclosure;
FIG. 2 is a perspective view, partially in section, of a patient, with a suction device of the system placed against the skin over a cellulite treatment site on the patient and a cannula on an enzyme injection device of the system inserted through an incision in the skin adjacent to or nearby the cellulite treatment site in typical implementation of the cellulite eradication methods;
FIG. 3 is a sectional view of the suction device, deployed in place against the skin of the patient over the cellulite treatment site, and an incision made in the skin adjacent or close to the cellulite treatment site, preparatory to external application of negative pressure to the skin through the suction device;
FIG. 4 is a sectional view of the suction device, maintained in place against the skin of the patient over the cellulite treatment site as negative pressure is externally applied to the skin through the suction device to form a suctioned skin area as the cannula of the enzyme injection device, inserted through the incision, is vibrated or reciprocated in the subcutaneous space beneath the cellulite treatment site during injection of a collagen degrading enzyme through the cannula into the subcutaneous space beneath the skin;
FIG. 5 is a cross-sectional view of the patient's skin, more particularly illustrating initial formation of the incision in a first treatment area of the patient's skin to be treated at the cellulite treatment site;
FIG. 6 is a cross-sectional view of the patient's skin with external application of negative pressure to the surface of the skin at the first treatment area maintained preparatory to insertion of the cannula (not illustrated) of the enzyme injection device through the incision in the skin and into the subcutaneous space;
FIG. 7 is a cross-sectional view of the patient's skin with the cannula of the enzyme injection device inserted through the incision into the subcutaneous space at the first treatment area as the suction device continues to externally apply negative pressure to the surface of the skin, the cannula is vibrated to internally apply positive pressure inside the skin and the collagen degrading enzyme is injected through the cannula into the subcutaneous space;
FIG. 8 is a cross-sectional view of the patient's skin preparatory to treatment of a second treatment area at the cellulite treatment site on the patient's skin after completion of the treatment at the first treatment area in the cellulite treatment site;
FIG. 9 is a cross-sectional view of the patient's skin illustrating the cannula of the enzyme injection device inserted through the incision into the subcutaneous space at the second treatment area with simultaneous external application of negative pressure to the surface of the skin, vibration of the cannula and injection of the collagen degrading enzyme through the cannula into the subcutaneous space;
FIG. 10 is a cross-sectional view of the patient's skin upon conclusion of treatment;
FIG. 11 is a flow diagram of an illustrative embodiment of the cellulite eradication methods; and
FIG. 12 is a flow diagram of an alternative illustrative embodiment of the cellulite eradication methods.

### DETAILED DESCRIPTION

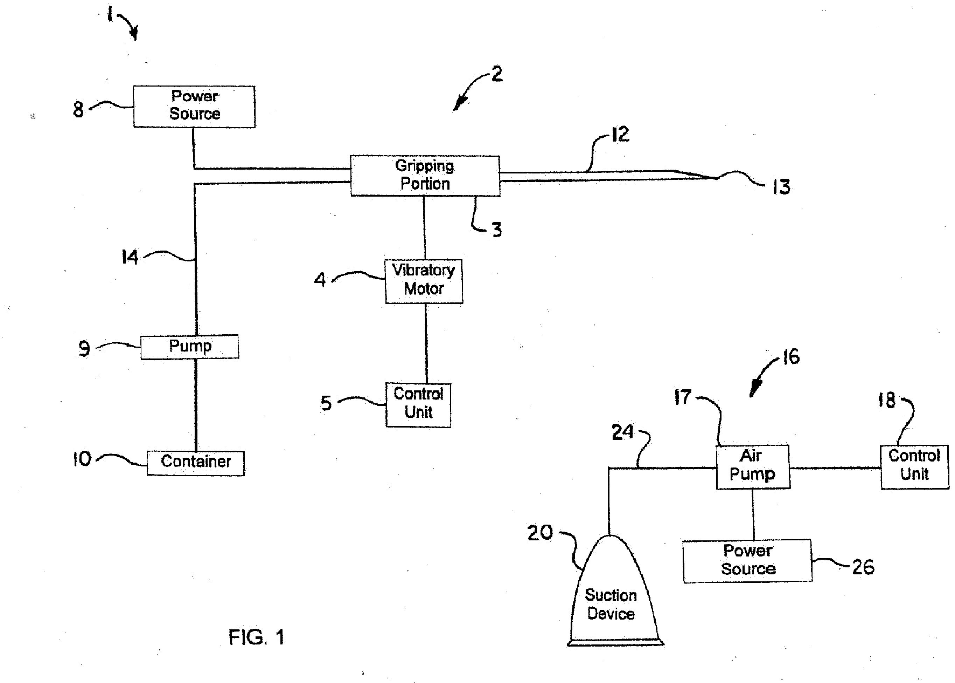

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper", "lower", "left", "rear", "right", "front", "vertical", "horizontal", and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Illustrative embodiments of the disclosure are generally directed to cellulite eradication methods in which a collagen degrading enzyme may be injected into a subcutaneous space to target, degrade and release cellulite band septa which extend between the fascia below and the dermis above the subcutaneous space. In some embodiments, the cellulite eradication methods may include injecting a collagen degrading enzyme into the subcutaneous space and into contact with the cellulite band septa.

In some embodiments, the cellulite eradication methods may further include vibrating the collagen degrading enzyme as the enzyme is injected into the subcutaneous space.

In some embodiments, the cellulite eradication methods may further include externally applying negative air pressure to at least one treatment area within the cellulite treatment site as the collagen degrading enzyme is injected into the subcutaneous space.

In some embodiments, the cellulite eradication methods may include externally applying negative air pressure to at least one treatment area within a cellulite treatment site and simultaneously injecting a collagen degrading enzyme into the subcutaneous space in the skin at the at least one treatment area while vibrating the collagen degrading enzyme.

In some embodiments, the cellulite eradication methods may include forming an incision adjacent or close to a cellulite treatment site on the skin of a patient, inserting a cannula of an enzyme injection device through the incision and into an adipose tissue layer in the subcutaneous space in the skin, injecting a collagen degrading enzyme through the cannula into the subcutaneous space into contact with cellulite band septa extending between the fascia and the dermis in the subcutaneous space, removing the cannula from the incision and closing the incision.

In some embodiments, the cellulite eradication methods may include forming an incision in the skin adjacent or close to a cellulite treatment site on a patient, applying negative air pressure to at least one treatment area within the cellulite treatment site through a suction device, inserting a cannula of an enzyme injection device through the incision and into the adipose tissue layer in the subcutaneous space in the skin, vibrating the cannula, injecting a collagen degrading enzyme through the cannula into the subcutaneous space and into contact with the cellulite band septa, removing the cannula from the incision, removing the suction device from the skin and closing the incision.

In some embodiments, the enzyme injection device may include any type of positive-displacement pump which may be entirely hand-operated or manual, electromechanical, or both manual and electromechanical. For example and without limitations, in some embodiments, the enzyme injection device may include a syringe. The syringe may include a syringe barrel, a manual or electromechanical syringe plunger slidably disposed in the syringe barrel and a cannula extending from the syringe barrel.

As used herein, "collagen degrading enzyme" may include any type, number and combination of collagenases which are suitable for the purpose of at least partially cleaving or breaking the cellulite band septa after injection and upon contact of the collagenases with the cellulite band septa. For example and without limitation, in some applications, the collagen degrading enzyme may include the collagenase *clostridium histolyticum,* which is available under the trade names QWO®, XIAFLEX® and ZIAPEX®.

Referring initially to FIGS. 1 of the drawings, an illustrative embodiment of an external and internal expansion vibration injection system, hereinafter system, which is suitable for some implementations of the cellulite eradication methods of the disclosure is generally indicated by reference numeral 1. The system 1 may include an enzyme injection device 2. For example and without limitation, in some applications, the enzyme injection device 2 may have a design which is the same as or similar to that which is disclosed in U.S Pub. No. 2013/0310749, which is hereby incorporated by reference herein in its entirety. The enzyme injection device 2 may include a gripping portion 3. In some applications, at least one vibratory motor 4 may engage the gripping portion 3 for vibration. The vibratory motor 4 may be electric, hydraulic and/or pneumatic. For example and without limitation, in some applications, the vibratory motor 4 may have a design which is the same as or similar to those described with respect to the electric vibrational apparatuses or the pneumatic vibrational apparatuses which are disclosed in U.S. Pat. No. 10,173,000, which patent is hereby incorporated by reference herein in its entirety. A control unit 5 may operationally interface with the vibratory motor 4. The control unit 5 may include but is not limited to buttons, switches, slides, triggers, touch screens and/or other input control devices which are operable to control various operational parameters such as the vibration frequency and/or the vibration amplitude, for example and without limitation, of the vibratory motor 4 according to the knowledge of those skilled in the art. The control unit 5 may additionally include a display (not illustrated) which indicates such data as status information and/or vibrational intensity (rate and/or amplitude), for example and without limitation.

At least one power source 8 may operably interface with the gripping portion 3. The power source 8 may include at least one battery and/or at least one electrical wall outlet, for example and without limitation. The power source 8 may be operable to provide a source of electrical current to the vibratory motor 4 and control unit 5 through the gripping portion 3, as illustrated, or alternatively, directly to the vibratory motor 4 and/or the control unit 5.

An elongated cannula 12 may extend from the gripping portion 3. The cannula 12 may be coupled to the gripping portion 3 using a locked thread connector such as a Luer lock connector, for example and without limitation. In some embodiments of the enzyme injection device 2, the vibratory motor 4 may engage the cannula 12 for oscillation or reciprocation directly or indirectly through the gripping portion 3. The cannula 12 may include a trocar or any other type of needle which is typically used for fat or tissue injection procedures or the like and may have a sharpened, pointed or tapered cannula tip 13. In some applications, the cannula 12 may include an exploded-tip or expanded basket cannula such as those which are described in U.S. Pat. No. 10,188,280, which patent is hereby incorporated by reference herein in its entirety. A non-limiting example of a cannula 12 which is suitable for implementation of the methods is a 4-5 mm exploded tip cannula available from Surgistem Technologies of Boston, MA. In some applications, the cannula 12 may be angled to facilitate further "reach" of the cannula 12 during the enzyme injection procedure, resulting in more extensive and thorough equalization of enzyme in the patient's skin at the cellulite treatment site.

A pump 9 may be disposed in fluid communication with the gripping portion 3 such as through a suitable tubing 14. At least one canister or container 10 may be disposed in fluid communication with the pump 9. The pump 9 may include a peristaltic pump or any type of positive displacement pump or other automated and/or manual mechanical delivery system which is known by those skilled in the art and operable to pump a liquid, semisolid and/or solid material from the container 10 through the gripping portion 3 and the cannula 12, respectively. In some applications, the pump 9 may be configured to operate in both a suction (negative pressure) phase and an injection (positive pressure) phase. For example and without limitation, in some applications, the pump 9 may include a syringe such as a Toomey syringe known by those skilled in the art. In some embodiments, the pump 9 and the container 10 may be combined in the same device or apparatus. In some applications of the methods, the enzyme injection device 2 may include a syringe or other device which is suitable for the injection of liquids and may or may not have vibratory capability. For example and without limitation, the enzyme injection device 2 may include a syringe. The syringe may include a syringe barrel, a manual or electromechanical syringe plunger slidably disposed in the syringe barrel and a cannula extending from the syringe barrel.

As further illustrated in FIG. 1, the system 1 may further include a medical suction apparatus 16. The medical suction apparatus 16 may include an air pump 17. At least one power source 26 may operably interface with the air pump 17. The power source 26 may include at least one battery and/or at least one electrical wall outlet, for example and without limitation.

A control unit 18 may operationally interface with the air pump 17. The control unit 18 may be operable to control various operational parameters such as the suction strength and the suction and injection phases, for example and without limitation, of the air pump 17 according to the knowledge of those skilled in the art.

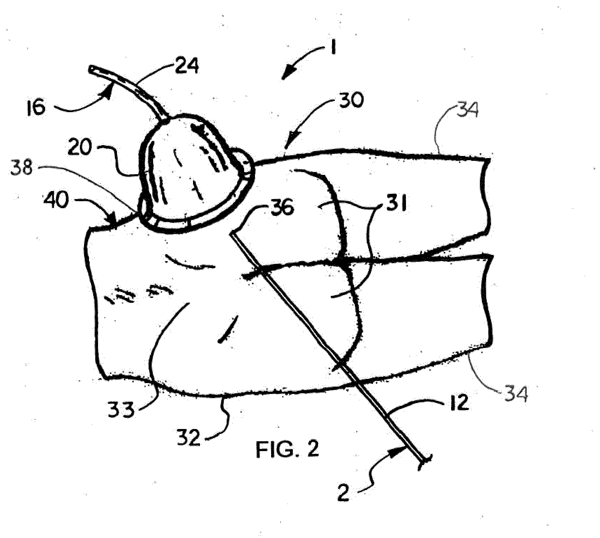

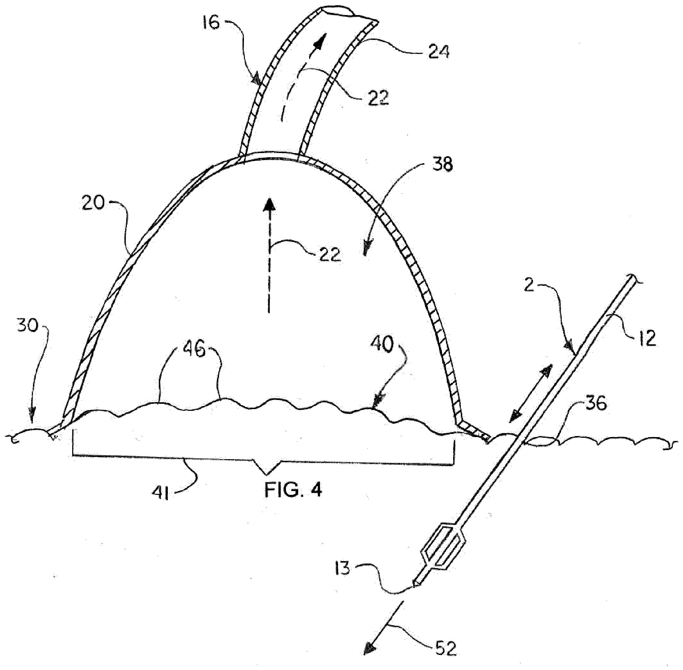

A suction device 20 may be disposed in pneumatic communication with the air pump 17 typically through suitable tubing 24. The suction device 20 may include a cup, dome or other concave structure which can be placed against a surface to impart a substantially airtight seal against the surface. Accordingly, in typical application of the system 1, as illustrated in FIGS. 2-4 and will be hereinafter further described, the suction device 20 may be placed against the skin 40 of a patient 30 to exert negative pressure 22 (FIG. 4) against the skin 40 responsive to operation of the air pump 17.

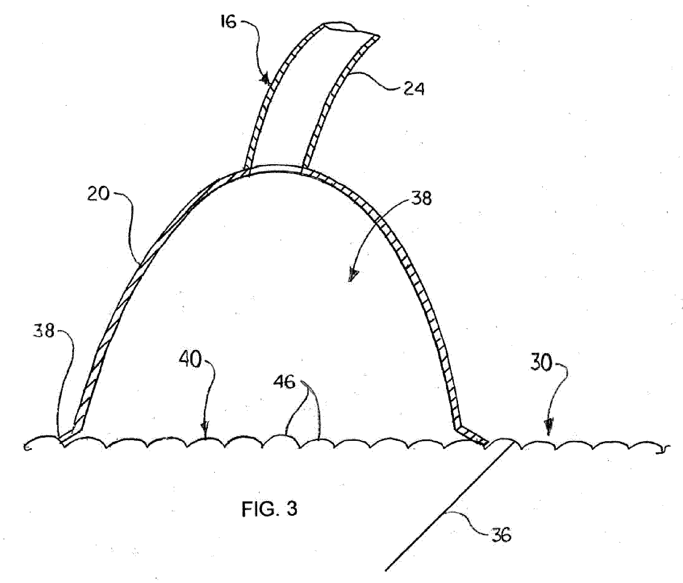

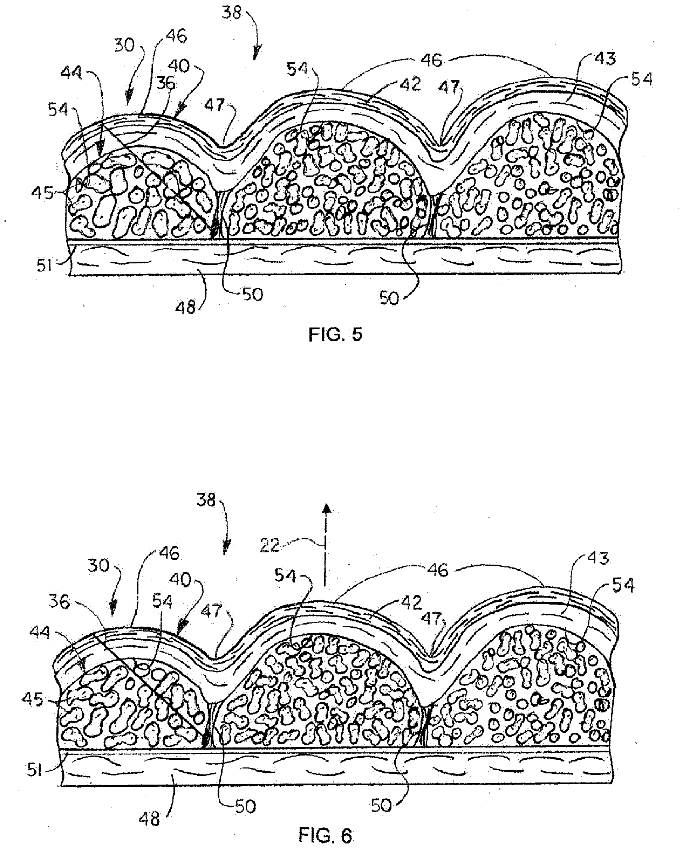

Referring next to FIGS. 2-10 of the drawings, in typical implementation of the cellulite eradication methods, the system 1 (FIG. 1) may be used to substantially eliminate or eradicate cellulite 46 from and fill out and smoothen the contour of the skin 40 of a patient 30. For example and without limitation, as illustrated in FIG. 2, the method may be implemented to substantially eradicate cellulite 46 (FIGS. 3 and 4) from the skin 40 in the buttocks 31, hip 32, lower back 33 and/or thighs 34 of the patient 30. As illustrated in FIG. 5, the skin 40 of the patient 30 has an epidermis 42 and an underlying dermis 43. An adipose tissue layer 44 having adipose cells 45 underlies the subdermal space beneath the dermis 43. A muscle layer 48 underlies the adipose tissue layer 44. A layer of fascia 51 overlies the muscle layer 48 beneath the adipose tissue layer 44.

Cellulite 46 in the skin 40 may be caused by deterioration of the dermis 43 due to a breakdown in blood vessel integrity and a loss of capillary networks in the dermis 43 and subdermal adipose tissue layer 44. This decreased metabolism hinders protein synthesis and repair processes, resulting in thinning of the dermis 43. Additionally, the adipose cells 45 in the adipose tissue layer 44 may become engorged with lipids, causing the adipose cells 45 to swell and clump together as well as retain excessive quantities of fluid in the adipose tissue layer 44.

As cellulite formation progresses, reticular protein deposits may form cellulite band septa 50 between the fascia 51 and the dermis 43 in the adipose tissue layer 44. Eventually, the cellulite band septa 50 may contract and harden (schleroses) as the spaces, or lobuli 54, within the adipose tissue layer 44 expand. Consequently, the thickening adipose tissue layer 44 causes the lobuli 54 to bulge upwardly between and form cellulite dimples 47 at the attachment points of the respective cellulite band septa 50 with the dermis 43. This effect results in the characteristic "orange peel" or "cottage cheese" appearance of cellulite 46 on the skin 40.

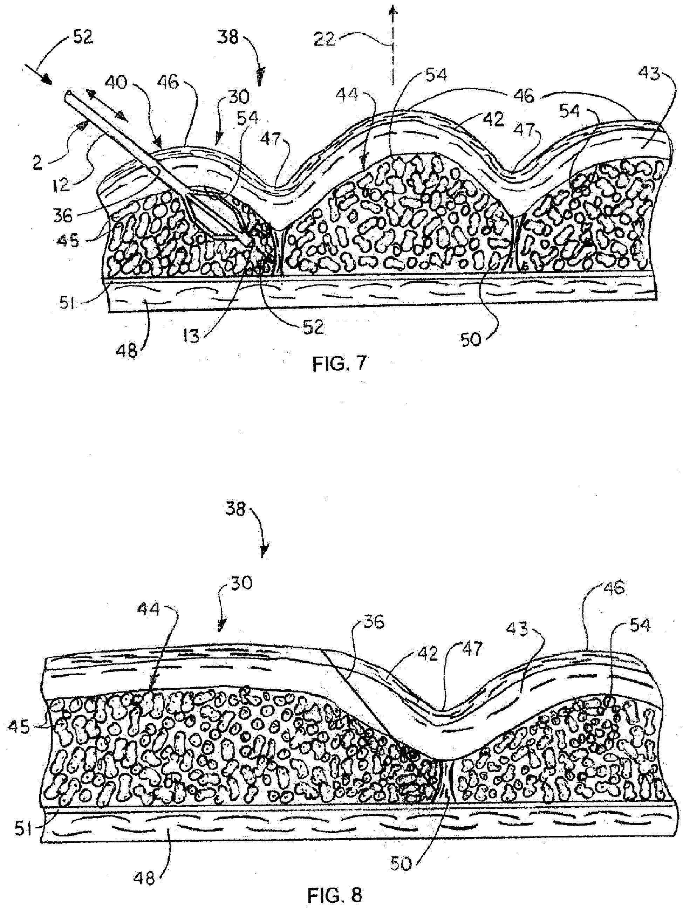

In the non-limiting example illustrated in FIGS. 5-10, the methods may be implemented to substantially eradicate or eliminate the cellulite 46 from the skin 40 to volumetrically enhance, augment, fill out or render uniform the contour of the skin 40 at the cellulite treatment site 38. Accordingly, in some implementations of the method, a sufficient volume of a collagen degrading enzyme 52 (FIG. 7) which will subsequently be injected into the skin 40 at the cellulite treatment site 38 may initially be placed in the container 10 (FIG. 1) of the enzyme injection device 2. The collagen degrading enzyme 52 may facilitate breakage or cleavage of at least a portion of the cellulite band septa 50 to at least partially eradicate or eliminate the cellulite 46 from the skin 40 throughout the cellulite treatment site 38.

The collagen degrading enzyme 52 may include any type, number and combination of collagenases which are suitable for the purpose of at least partially cleaving or breaking the cellulite band septa 50 after injection and upon contact of the collagenases with the cellulite band septa 50. For example and without limitation, in some applications, the collagen degrading enzyme 52 may include the collagenase *clostridium histolyticum,* which is available under the trade names QWO®, XIAFLEX® and ZIAPEX® and is commonly used in the treatment of Dupuytren's contracture. The injection cannula 12 of the enzyme injection device 2 may be used to facilitate targeted release of the cellulite band septa 50 and at least partially eradicate or eliminate the cellulite 46 from the skin 40, resulting in smoothening of the contour of the skin 40 at the cellulite treatment site 38.

As illustrated in FIG. 2, as the patient 30 typically lies in a prone or lateral position, after infiltration of local anesthetic, an incision 36 may be formed in the skin 40 adjacent to the cellulite treatment site 38. As illustrated in FIG. 5, the incision 36 may extend through the epidermis 42 and the dermis 43 and into the underlying adipose tissue layer 44.

As illustrated in FIGS. 2 and 3, the suction device 20 on the medical suction apparatus 16 of the system 1 (FIG. 1) may be placed against the surface of the skin 40 over at least one treatment area within the cellulite treatment site 38 to achieve an airtight seal between the suction device 20 and the skin 40. The air pump 17 (FIG. 1) of the medical suction apparatus 16 may be operated to externally apply negative pressure 22 to the surface of the skin 40 through the tubing 24 and the interior of the suction device 20. As illustrated in FIG. 4, the negative pressure 22 may form a raised or convex suctioned skin area 41 which substantially conforms to the size and shape of the suction device 20 on the surface of the skin 40 at the cellulite treatment site 38. As illustrated in FIG. 6, the suctioned skin area 41 (FIG. 4) may include the epidermis 42 and the dermis 43 and may further include at least a portion of the underlying adipose layer 44. Throughout the cellulite treatment site 38, the negative pressure 22 may apply tensile tension to the cellulite band septa 50.

As illustrated in FIG. 7, as the suction device 20 (FIGS. 2-4) is maintained in place on the skin 40, the cannula 12 of the enzyme injection device 2 may be inserted through the incision 36 until the cannula tip 13 of the cannula 12 terminates in the adipose tissue layer 44. The pump 9 (FIG. 1) of the tissue injection device 2 may be operated in the injection phase to pump the collagen digesting enzyme 52 from the container 10 through the tubing 14, gripping portion 2 and cannula 12, respectively, and into the adipose tissue layer 44. In areas of excessive cellulite band septa 50, flow of the collagen degrading enzyme 52 may be reduced or halted temporarily while the exploded-tip cannula 12 is manipulated to loosen or release the cellulite band septa 50 and diminish resistance in tight areas. A typical volume of collagen digesting enzyme 52 which may be injected may range from 0.25 cc to 10 cc, depending typically on the number of cellulite lesions and concentration of collagenase.

In some applications, the vibratory motor 4 (FIG. 1) of the tissue injection device 2 may simultaneously be operated to vibrate or reciprocate the injection cannula 12 as the collagen degrading enzyme 52 is injected into the adipose tissue layer 44. The vibratory motion of the injection cannula 12 may achieve enhanced dispersal and homogenous distribution of the collagen degrading enzyme 52 throughout the area or areas of the cellulite band septa 50 as well as facilitate positive internal pressure which augments the outward expansion of the epidermis 42 and the dermis 43 facilitated by the externally-applied negative pressure 22. The various operational parameters of the vibratory motor 4, such as the vibration frequency and/or the vibration amplitude, for example and without limitation, may be selected by input from the control unit 5. A typical vibration or reciprocation frequency for the injection cannula 12 may fall within the range of about 2,000-10,000 cycles/min, with a typical frequency of about 7,000 cycles/min. A typical time period over which the injection cannula 12 may be vibrated during injection may range from several seconds up to several minutes, depending typically on the number and severity of cellulite lesions.

In some applications, the expanded basket cannula 12 may be used in conjunction with the vibratory motion of the cannula 12 to further facilitate internal positive expansion within the adipose tissue layer 44 in conjunction with the external negative expansion which is applied by the negative pressure 22 on the surface of the skin 40. Accordingly, the expanded basket cannula 12 may create differential high- and low-pressure zones in the adipose tissue layer 44 such that the adipose cells 45 migrate from the high-pressure zones to the low-pressure zones. Consequently, the internal positive pressure applied by the vibrating cannula 12, in conjunction with the external negative pressure applied by the suction device 20, may increase the tensile force which is applied to the cellulite band septa 50 as well as the number of cellulite band septa 50 which are tensioned throughout the treatment area or areas within the cellulite treatment site 38. Tensioning of the cellulite band septa 50 may enhance the capability of the collagen degrading enzyme 52 to break, cleave or release the cellulite band septa 50 as the collagen degrading enzyme 52 is discharged from the cannula tip 13 of the cannula 12 into the adipose tissue layer 44.

As illustrated in FIG. 8, after the cellulite band septa 50 in a first treatment area within the cellulite treatment site 38 are cleaved, the injection cannula 12 may be removed from the incision 36, the suction device 20 removed from the skin 40 and the incision 36 closed. In some applications of the methods, another incision 36 may be made in the skin 40 at a second treatment area within the cellulite treatment site 38. In other applications of the methods, the same incision 36 may be used for insertion of the cannula 12 in multiple treatment areas within the cellulite treatment site 38. For example and without limitation, for a cellulite treatment site 38 having a relatively small area, one incision 36 may be sufficient to provide an access for the cannula 12. In applications in which the cellulite treatment site 38 has a larger area on the skin 40 of the patient 30, multiple incisions 36 may be required to facilitate positioning of the cannula 12 in the different treatment areas of the cellulite treatment site 38. The suction device 20 (FIGS. 2-4) may again be applied against the skin 40 of the patient 30 at the second treatment area.

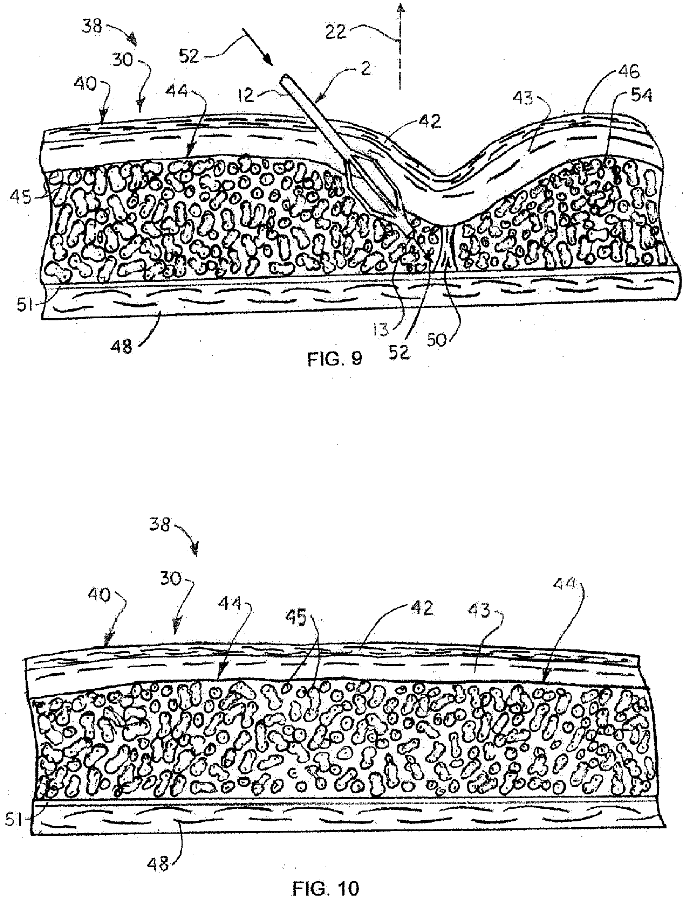

As illustrated in FIG. 9, as the suction device 20 (FIGS. 2-4) is maintained in place on the skin 40 at the second treatment area within the cellulite treatment site 38, the cannula 12 of the enzyme injection device 2 may be inserted through the incision 36 at the second treatment area until the cannula tip 13 of the cannula 12 terminates in the adipose tissue layer 44. The pump 9 (FIG. 1) of the tissue injection device 2 may be operated in the injection phase to pump the collagen digesting enzyme 52 from the container 10 through the tubing 14, gripping portion 2 and cannula 12, respectively, and into the adipose tissue layer 44. The discharged collagen digesting enzyme 52 may break, cleave or release the cellulite band septa 50 throughout the second treatment area. The cannula 12 may then be withdrawn from the incision 36 and the incision 36 closed, as was heretofore described.

Multiple injections may be necessary per treatment, depending typically on the number, proximity and severity of the cellulite lesions. Accordingly, the same procedure which was heretofore described with respect to FIGS. 5-9 may be repeated until the cellulite 46 throughout the cellulite treatment site 38 is substantially eradicated. Accordingly, as illustrated in FIG. 10, after recovery, the treated skin 40 has a reshaped contour and substantially lacks the cellulite 46 (FIG. 5), enhancing the aesthetic appearance of the skin 40 to the satisfaction of the patient 30. Full activity on the part of the patient may be recovered typically within 2-3 days after treatment, resulting in typically 50-100% reduction in the cellulite lesions after a single treatment.

It will be appreciated by those skilled in the art that the cellulite eradication methods of the disclosure may enable a surgeon to easily place a collagen degrading enzyme at selected quantities and in selected areas at a cellulite treatment site in the adipose tissue layer of a patient to facilitate targeted release of cellulite band septa and substantial eradication of cellulite. The result is enhanced control over contouring and smoothening of the skin in the affected areas. The % eradication may be measured using the Physician-graded global aesthetic improvement scale (GAIS) assessment.

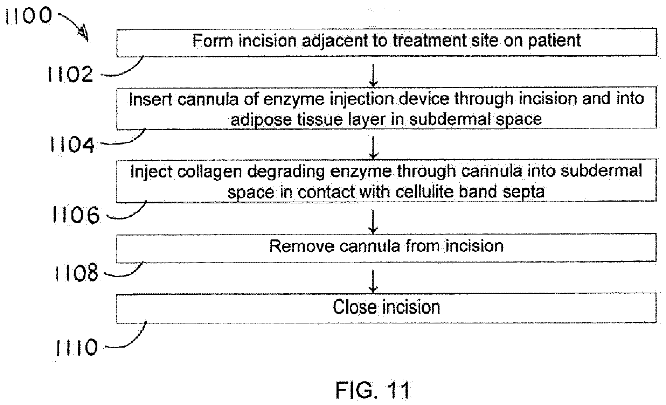

Referring next to FIG. 11 of the drawings, a flow diagram 1100 of an illustrative embodiment of the cellulite eradication methods is illustrated. The methods may include initially forming an incision in the skin of a patient adjacent to a cellulite treatment site at Step 1102.

At Step 1104, a cannula of an enzyme injection device may be inserted through the incision and into a subcutaneous adipose tissue layer.

At Step 1106, a collagen degrading enzyme may be injected through the cannula into the adipose tissue layer and in contact with cellulite band septa.

At Step 1108, the cannula may be removed from the incision.

At Step 1110, the incision may be closed. Steps 1102-1110 may be repeated at other treatment areas at the cellulite treatment site as deemed necessary to substantially eradicate cellulite from the cellulite treatment site.

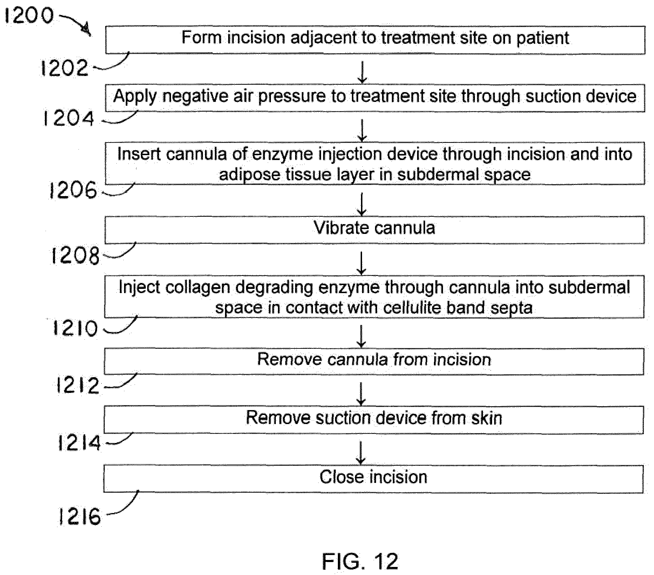

Referring next to FIG. 12 of the drawings, a flow diagram 1200 of an alternative illustrative embodiment of the cellulite eradication methods is illustrated. At Step 1202, an incision may be formed in the skin of a patient adjacent to a cellulite treatment site.

At Step 1204, negative air pressure may be externally applied to the cellulite treatment site through a suction device.

At Step 1206, a cannula of an enzyme injection device may be inserted through the incision and into an adipose tissue layer in the subcutaneous space.

At Step 1208, the cannula may be vibrated.

At Step 1210, a collagen degrading enzyme may be injected through the cannula into the subcutaneous space and into contact with cellulite band septa.

At Step 1212, the cannula may be removed from the incision.

At Step 1214, the suction device may be removed from the skin of the patient.

At Step 1216, the incision may be closed.

The following examples are intended to illustrate the invention, and should not be construed as limiting the invention in any way.

### EXAMPLE 1

A 30-year-old female patient with moderate cellulite on the thighs was placed in a prone operative position. The cellulite treatment site on the patient was anesthetized with local anesthetic, after which an incision was made at the treatment site through the epidermis and dermis and into the underlying adipose tissue layer. The suction device on the medical suction apparatus of the external and internal expansion vibration injection system was placed against the surface of the skin at the cellulite treatment site to achieve an airtight seal between the suction device and the skin. The air pump of the medical suction apparatus was operated to externally apply negative pressure to the surface of the skin through the suction device. The negative pressure formed a raised or convex suctioned skin area on the surface of the skin at the cellulite treatment site and applied tensile tension to the cellulite band septa in the skin. An exploded tip injection cannula on the enzyme injection device was inserted through the incision until the cannula tip of the cannula terminated in the adipose tissue layer beneath the suctioned skin area. The pump of the tissue injection device was then operated in the injection phase to pump 5 cc of *clostridium histolyticum* (XIAFLEX®) collagenase enzyme from the container through the exploded tip cannula and into the adipose tissue layer. Simultaneous with injection of the collagenase, the vibratory motor of the tissue injection device was operated to vibrate the injection cannula for a period of 3 minutes at a vibration frequency of 7,000 cycles/min. The injection cannula was withdrawn from the incision, the incision closed and standard post-operative procedures followed. The patient returned to full activity 3 days after the procedure, and a 95% eradication of the cellulite lesions (for example as may be measured using the Physician- graded global aesthetic improvement scale (GAIS) assessment) was achieved.

While certain illustrative embodiments of the disclosure have been described above, it will be recognized and understood that various modifications can be made to the embodiments and the appended claims are intended to cover all such modifications which may fall within the spirit and scope of the disclosure.

## Claims

1. A cellulite eradication method for substantially eradicating cellulite (46) from a cellulite treatment site on the skin (40) of a patient (30), comprising:
injecting a collagen degrading enzyme (52) into a subcutaneous space and into contact with cellulite band septa (50) in the skin (40) of the patient (30).

2. The cellulite eradication method of claim 1 wherein injecting a collagen degrading enzyme (52) into the subcutaneous space comprises injecting the collagenase *clostridium histolyticum* into the subdermal space.

3. The cellulite eradication method of claim 1 or 2 further comprising vibrating the collagen degrading enzyme (52) simultaneous with injecting the collagen degrading enzyme (52) into the subcutaneous space, preferablywherein vibrating the collagen degrading enzyme (52) comprises vibrating the collagen degrading enzyme (52) at a reciprocation frequency within the range of about 2,000-10,000 cycles/min, more preferably at a reciprocation frequency of about 7,000 cycles/min.

4. The cellulite eradication method of any one of claims 1 to 3 further comprising externally applying negative air pressure to at least one treatment area within the cellulite treatment site simultaneous with injecting the collagen degrading enzyme (52) into the subcutaneous space.

5. The cellulite eradication method of any one of claims 1 to 4 wherein injecting the collagen degrading enzyme (52) into the subcutaneous space comprises injecting the collagen degrading enzyme (52) into the subcutaneous space to substantially eradicate cellulite (46) from the skin (40) in at least one of the buttocks (31), hip (32), thighs (34), and lower back (33) of the patient (30).

6. A cellulite eradication method for substantially eradicating cellulite (46) from a cellulite treatment site on the skin (40) of a patient (30), comprising:
forming an incision (36) adjacent or close to the cellulite treatment site (38) on the skin (40) of the patient (30);
inserting a cannula (12) of an enzyme injection device (2) through the incision (36) and into an adipose tissue layer (44) in the subcutaneous space in the skin (40);
injecting a collagen degrading enzyme (52) through the cannula (12) into the subcutaneous space into contact with cellulite band septa (50) extending between the fascia (51) and the dermis (43) in the subcutaneous space;
removing the cannula (12) from the incision (36); and
closing the incision (36).

7. The cellulite eradication method of claim 6 wherein injecting the collagen degrading enzyme (52) into the subcutaneous space comprises injecting the collagenase *clostridium histolyticum* into the subcutaneous space.

8. The cellulite eradication method of claim 6 or 7 further comprising vibrating the collagen degrading enzyme (52) simultaneous with injecting the collagen degrading enzyme (52) into the subcutaneous space, preferably wherein vibrating the collagen degrading enzyme (52) comprises vibrating the collagen degrading enzyme (52) at a reciprocation frequency within the range of about 2,000-10,000 cycles/min, more preferablyat the reciprocation frequency of about 7,000 cycles/min.

9. The cellulite eradication method of any one of claims 6 to 8 further comprising externally applying negative air pressure to at least one treatment area within the cellulite treatment site simultaneous with injecting the collagen degrading enzyme (52) into the subcutaneous space.

10. The cellulite eradication method of any one of claims 6 to 9 wherein injecting the collagen degrading enzyme (52) into the subcutaneous space comprises injecting the collagen degrading enzyme (52) into the subcutaneous space to substantially eradicate cellulite (46) from the skin (40) in at least one of the buttocks (31), thighs (34), hip (32) and lower back (33) of the patient (30).

11. A cellulite eradication method for substantially eradicating cellulite (46) from a cellulite treatment site on the skin (40) of a patient (30), comprising:
obtaining an external and internal expansion vibration lipofilling system including an enzyme injection device (2) having a cannula (12) with a cannula tip (13) and a medical suction apparatus (16) having a suction device (20);
forming an incision (36) adjacent to the cellulite treatment site on the skin (40) of the patient (30);
externally applying negative air pressure to at least one treatment area within the cellulite treatment site through the suction device (20) of the medical suction apparatus (16);
inserting the cannula (12) of the enzyme injection device (2) through the incision (36) and into an adipose tissue layer (44) in the subcutaneous space in the skin (40);
injecting a collagen degrading enzyme (52) through the cannula (12) into the subcutaneous space into contact with cellulite band septa (50) extending between the fascia (51) and the dermis (43) in the subcutaneous space;
removing the cannula (12) from the incision (36); and
closing the incision (36).

12. The cellulite eradication method of claim 11 wherein injecting the collagen degrading enzyme (52) into the subcutaneous space comprises injecting the collagenase *clostridium histolyticum* into the subcutaneous space.

13. The cellulite eradication method of claim 11 or 12 further comprising vibrating the collagen degrading enzyme (52) simultaneous with injecting the collagen degrading enzyme (52) into the subcutaneous space, preferably wherein vibrating the collagen degrading enzyme (52) comprises vibrating the collagen degrading enzyme (52) at a reciprocation frequency within the range of about 2,000-10,000 cycles/min, more preferably
at the reciprocation frequency of about 7,000 cycles/min.

14. The cellulite eradication method of any one of claims 11 to 13 wherein injecting the collagen degrading enzyme (52) into the subcutaneous space comprises injecting the collagen degrading enzyme (52)into the subcutaneous space to substantially eradicate cellulite (46) from the skin (40) in at least one of the buttocks (31), thighs (34), hip (32) and lower back (33) of the patient (30).
